# EUROPÄISCHE PATENTSCHRIFT

(11) **EP 0 492 183 B1**
(45) Veröffentlichungstag und Bekanntmachung des Hinweises auf die Patenterteilung: **13.03.1996**
(21) Anmeldenummer: 91120700.9
(22) Anmeldetag: 02.12.1991
(51) Int. Cl.: A23L 1/305

(54) **Phenylalaninfreie Säuglings- und Kleinkindnahrungsgrundlage**
Phenylalanine-free food base for infants and small children
Base d'aliments exempts de phénylalanine pour nourrissons et petits enfants

(30) Priorität: 28.12.1990 DE 4042115
(43) Veröffentlichungstag der Anmeldung: 01.07.1992
(73) Patentinhaber: MILUPA AKTIENGESELLSCHAFT, D-61381 Friedrichsdorf (DE)
(72) Erfinder: Wachtel, Ursula, Dr., W-6380 Bad Homburg (DE); Schweikhardt, Friedrich, Dr., W-6382 Friedrichsdorf (DE); Tesmer, Erhard, Dr., W-6380 Bad Homburg (DE)
(74) Vertreter: Köster, Hajo, Dr.

(56) Entgegenhaltungen:
- EP-A- 0 053 985
- EP-A- 0 302 807
- DD-A- 157 258
- DE-A- 2 117 243

## Beschreibung

Die Erfindung betrifft eine phenylalaninfreie Säuglings- und Kindernahrungsgrundlage auf Basis einer Aminosäurenmischung, die alle für die Ernährung des Kindes erforderlichen L-Aminosäuren enthält, jedoch frei von Phenylalanin ist, sowie ein Verfahren zu deren Herstellung. Diese Nahrungsgrundlage ist für Kinder und Säuglinge bestimmt, die unter einer Phenylketonurie leiden.

Die Phenylketonurie (nachstehend PKU genannt) gehört zu den genetisch bedingten Erkrankungen und stellt eine Stoffwechselstörung dar.

Phenylalanin wird unter normalen Ernährungsbedingungen mit allen Proteinen tierischer und pflanzlicher Herkunft aufgenommen. Nach der Verdauung der nativen Proteine und Resorption der Spaltprodukte gelangt Phenylalanin über den Aminosäuren-Pool in die Leber und wird dort in einer irreversiblen Reaktion durch das Enzym Phenylalaninhydroxylase zu Tyrosin hydroxyliert. Bei Säuglingen und Kleinkindern wird in Folge des raschen Wachstums ein Teil des Phenylalanins für die körpereigene Proteinsynthese benötigt.

Als Folge der Stoffwechselstörung häuft sich bei den erkrankten Kindern das Phenylalanin im Körper an, so daß sich dessen Gehalt im Blut und im Gewebe weit über den Normalbereich hinaus erhöht.

Der erhöhte Phenylalaninspiegel im Plasma führt zu einer Beeinträchtigung zahlreicher Stoffwechselvorgänge im Gehirn. Wird die PKU nicht behandelt, kommt es bei Kindern zu einer Hirnreifestörung mit geistigem Entwicklungsrückstand in unterschiedlicher Ausprägung. Da sich das Gehirn in den ersten Lebensmonaten außerordentlich rasch entwickelt und dabei sehr störanfällig ist, muß die Erkrankung so frühzeitig als möglich erkannt und dann konsequent behandelt werden.

Um bei einem unter PKU leidenden Kind eine normale geistige und körperliche Entwicklung zu erzielen, muß sein Phenylalaninspiegel im Plasma mit einer phenylalaninarmen Diät auf Normalwerte gesenkt und stabilisiert werden.

Dazu bekommen derartige Kinder eine Nahrung, die eine beschränkte Menge an natürlichem Eiweiß und gerade soviel Phenylalanin enthält, wie der kindliche Körper zum Aufbau von Eiweiß (Wachstum) benötigt. Für die Ernährung können daher nur solche Lebensmittel eingesetzt werden, die von Natur aus einen niedrigen Eiweißgehalt und damit auch einen niedrigen Phenylalaningehalt besitzen.

Mit einer derartigen phenylalaninarmen Ernährung allein würde den Kindern jedoch zu wenig von allen anderen, für das Leben ebenso wichtigen Aminosäuren zugeführt werden. Daher benötigen Kinder, die an der PKU leiden, zusätzlich zu einer phenylalaninarmen Diät eine Eiweißquelle, die zwar kein Phenylalanin, jedoch aber eine ausreichende Menge aller anderen Aminosäuren enthält.

Es sind bereits Spezialerzeugnisse bekannt, die aus Eiweißbausteinen, den Aminosäuren, zusammengesetzt sind, jedoch kein Phenylalanin enthalten. Diesen Mischungen sind zusätzlich Vitamine, Mineralstoffe und Spurenelemente einverleibt, da ein unter der PKU leidendes Kind diese Nährstoffe mit der phenylalaninarmen Ernährung allein nicht in ausreichenden Mengen erhalten würde. Diese bekannten Spezialerzeugnisse sind aus Mischungen von L-Aminosäuren oder von speziellen extrem phenylalaninarmen Eiweiß- bzw. Proteinhydrolysaten aufgebaut.

Die Ernährung eines an der PKU leidenden Säuglings besteht nun aus einer Flaschennahrung, die sich aus adaptierter Säuglingsmilchnahrung und/oder Muttermilch und dem bekannten Spezialerzeugnis bzw. der bekannten phenylalaninfreien Aminosäurenmischung zusammensetzt. Um den Energiebedarf des Säuglings zu decken, müssen dieser Nahrung noch Energieträger hinzugefügt werden, z.B. Traubenzucker, Stärke und pflanzliche Öle. Letztere müssen auch zur Versorgung mit der essentiellen Linolsäure beitragen.

Die Zubereitung der Flaschennahrung für den Säugling erfolgte bisher in der Klinik oder durch die Mutter zuhause auf äußerst aufwendige Weise. Es ist dabei erforderlich, mehrere Nährstoffe und Produkte zusammenzubringen, um eine Diät für das unter der Phenylketonurie leidende Kind bereitzustellen.

Die Wachstumsgeschwindigkeit eines Säuglings vermindert sich jedoch im ersten Lebensjahr sehr rasch. Dies bedeutet auch, daß sich der Bedarf an Phenylalanin in ganz kurzen Zeitabständen ändert. Demzufolge muß auch die Zusammensetzung der Flaschennahrung ständig neu berechnet werden. In einer Studie unter Beteiligung von acht Universitätskliniken hat sich nun gezeigt, daß die Mütter mit dieser Berechnung der Diät völlig überfordert sind. Sie können die erforderlichen komplizierten Energie- und Nährwertberechnungen nicht selbst durchführen. Jede Flaschenzubereitung besteht aus mindestens sechs Komponenten, die nach individuellem Diätplan zu berechnen, abzuwägen, zu kochen bzw. zu mischen sind.

Mit Aminosäuren angereicherte Nahrungsprodukte sind im Stand der Technik bereits bekannt. So beschreibt die EP-A 0 302 807 mit Nukleosiden und/oder Nukleotiden angereicherte Nahrungsprodukte für Kinder und Erwachsene. Bei der Herstellung dieser Nahrung wird unter anderem die Sprühtrocknung als reines Trocknungsverfahren eingesetzt. Bei diesem bekannten Nahrungsprodukten handelt es sich um fertige, als solche zu genießende Nahrungsmittel.

Ferner ist es aus der DE-A 217 243 bekannt, zur Herstellung von Trockenprodukten aus wässrigen Lösung eine Walzentrocknung von phenylalaninarmen Eiweißhydrolysaten vorzunehmen, um ein geschmacksneutrales und wohlschmeckendes, phenylalaninarmes Eiweißhydrolysat für die Ernährung von an der Phenylketonurie leidenden Säuglingen und Kindern bereitzustellen.

Aufgabe der vorliegenden Erfindung ist es, eine phenylalaninfreie Säuglings- und Kleinkindernahrungsgrundlage bereitzustellen, welche die Berechnung und Zubereitung einer Flaschennahrung für an der PKU leidende Säuglinge und Kleinkinder so einfach als möglich gestaltet, so daß diätetische Fehler vermieden werden.

Gelöst wird diese Aufgabe durch die Lehre des Anspruchs 1.

Die erfindungsgemäße phenylalaninfreie Nahrungsgrundlage enthält außer den Aminosäuren noch Vitamine, Mineralstoffe, Spurenelemente, Energielieferanten (Zucker, Stärke) und essentielle Fettsäuren und ist mit adaptierter Säuglingsmilchnahrung in jedem Verhältnis mischbar.

Bei Verwendung der erfindungsgemäßen Nahrungsgrundlage entfällt die Nährwertberechnung sowie die Zubereitung von fünf der insgesamt sechs Komponenten, wie das bei der Herstellung der bisher verwendeten Nahrung der Fall war. Die erfindungsgemäße Nahrungsgrundlage wird nur noch mit adaptierter Säuglingsmilchnahrung gemischt, welche die individuelle Toleranz an Phenylalanin abdeckt.

Die Verwendung der erfindungsgemäßen Nahrungsgrundlage stellt für die betroffenen Mütter eine außerordentliche Erleichterung bei der praktischen Durchführung der PKU-Diät dar und wirkt sich positiv auf das betroffene Kind aus. Die Möglichkeit einer fehlerhaften Zubereitung der Nahrung für das Kind mit den Auswirkungen auf seine Gehirnentwicklung ist außerordentlich gering. Zudem kann die Zusammensetzung der erfindungsgemäßen Nahrungsgrundlage so gewählt werden, daß sich diese und die adaptierte Säuglingsmilchnahrung im Angebot an Energie und essentiellen Nährstoffen gegenseitig ergänzen, so daß eine Bedarfsdeckung ähnlich der aus Frauenmilch resultiert.

Die Aminosäuren können in der erfindungsgemäßen Nahrungsgrundlage in jeder geeigneten Form insbesondere in jeder für Lebensmittelzwecke zulässigen Form vorliegen. So können die Aminosäuren beispielsweise als Salze, Hydrochloride, Hydrate, Acetate und Malate. vorliegen. Sie können ferner in Form von Glutamaten und Aspartaten Anwendung finden. Auch ist es möglich, die Aminosäuren in Form von Dipeptiden zur Anwendung zu bringen, sofern diese Dipeptide kein Phenylalanin enthalten.

Vorzugsweise werden die Aminosäuren jedoch vorwiegend als freie Aminosäuren eingesetzt. Das Lysin wird vorzugsweise als Lysinglutamat zur Anwendung gebracht.

Bei der erfindungsgemäßen Nahrungsgrundlage ist das Aminosäuremuster vorzugsweise demjenigen in adaptierten herkömmlichen Nahrungen und/oder in der Frauenmilch angeglichen, wobei natürlich das Phenylalanin aus der Rezeptur weggelassen wird.

Als Fette können solche unterschiedlicher Herkunft (tierisch oder pflanzlich) und auch mittelkettige Triglyceride (MCT-Fette) in wechselnden Mischungsverhältnissen eingesetzt werden, wobei der Gehalt an Fetten vorzugsweise zwischen 10 und 30 % liegt, bezogen auf die Trockenmasse des Gesamtprodukts.

Auch die Kohlenhydrate können verschiedener Herkunft und unterschiedlicher Zusammensetzung sein. So können beispielsweise Mono-, Di-, Oligo- und Polysacharide (auch Stärken) eingesetzt werden, wobei die Kohlenhydrate vorzugsweise bis zu 70 % ausmachen, bezogen auf die Trockenmasse des Produkts.

Die Mineralstoffe und Spurenelemente können in wechselnden Mengen zugesetzt werden, um das Produkt gemäß den pädiatrischen Empfehlungen und Richtlinien für Säuglings- und Kleinkindernahrungen mit allen essentiellen Mineralstoffen und Spurenelementen anzureichern und zu standardisieren.

Die erfindungsgemäße Nahrungsgrundlage kann auch gemäß den genannten Empfehlungen und Richtlinien vitaminiert werden und mit wasser- und fettlöslichen Vitaminen in wechselnden Mengen versetzt werden.

Die erfindungsgemäße Nahrungsgrundlage ist vorzugsweise dadurch erhältlich, daß man alle Inhaltsstoffe (bis auf die Vitamine) dieser Nahrungsgrundlage in Wasser emulgiert, dispergiert und löst. Den so hergestellten Naßansatz homogenisiert, pasteurisiert und sprühtrocknet man. Das Sprühprodukt wird dann gegebenenfalls mit einer Vitaminmischung und einem Rest Laktoseanteil vermischt. Das erhaltene, gemischte Produkt wird als gebrauchsfertiges Pulver abgepackt.

Es hat sich gezeigt, daß es bei der Herstellung des Sprühproduktes von besonderer Bedeutung ist, zunächst Fett, Emulgatoren und Wasser vorzuemulgieren, insbesondere bei 70 bis 75°C. Gegebenenfalls homogenisiert man dann mit hohem Druck.

Ferner ist es von Vorteil, im nächsten Schritt alle Kohlenhydrate in den Naßansatz einzuarbeiten, um eine Anhebung der Viskosität des Ansatzes zu erreichen. Die anschließende Zugabe der Mineralstoffe und Spurenelemente schafft eine günstige Ionenkonzentration und ein entsprechendes pH-Milieu für die Einarbeitung der L-Aminosäuren.

Freie Aminosäuren stören die Emulsionsstabilität. Es ist daher sinnvoll und bevorzugt, die Aminosäuren erst möglichst kurz vor der Fertigstellung des Naßansatzes zuzusetzen. Vorzugsweise kontrolliert man den pH-Wert des Naßansatzes und hält ihn bei 6,1 bis 6,3, wobei man gegebenenfalls mit Alkali- oder Erdalkalihydroxiden und/oder Karbonaten versetzt. Bei einer Verdünnung des Konzentrates bzw. des Naßansatzes auf 13 % Trockensubstanz sollte der pH-Wert des fertigen Ansatzes mindestens bei 6,6 liegen, um beim Mischen mit Säuglingsmilchnahrungen oder Muttermilch Eiweißausfällungen zu vermeiden.

Gegenstand der Erfindung ist auch ein Verfahren zur Herstellung einer phenylalaninfreien Säuglings- und Kleinkindernahrungsgrundlage.

Die oben gemachten und im Zusammenhang mit der Herstellung der erfindungsgemäßen Nahrungsgrundlage stehenden Ausführungen haben auch Gültigkeit für das erfindungsgemäße Verfahren.

Es hat sich nun gezeigt, daß die Einarbeitung der L-Aminosäuren in den Naßansatz in einer bestimmten Reihenfolge zweckmäßig ist, um während der Herstellung des Naßansatzes Ausfällungen und Polymerisationen zu verhindern. Die genannte Reihenfolge ist im weiter untenstehenden Beispiel näher erläutert.

Nachdem man die Aminosäuren in den Naßansatz eingebracht hat, führt man eine Homogenisierung durch, um mögliche beginnende Störungen der Emulsionsstabilität zu beseitigen. Anschließend pasteurisiert bzw. erhitzt man, zweckmäßigerweise mit einem Schabeerhitzer, wodurch eine starke Keimreduktion des Konzentrats bewirkt wird. Die anschließende Sprühtrocknung des Konzentrats bzw. des Naßansatzes führt man vorzugsweise in einem Sprühturm und weiterhin bevorzugt mit Düsen durch, da der Druckaufbau des Konzentrats vor den Düsen einen nochmaligen Homogenisiereffekt darstellt.

Die erfindungsgemäße Nahrungsgrundlage läßt sich zwar auch mit einer Sprühscheibe trocknen, doch sind die physikalischen Eigenschaften des Pulvers, wie Fließverhalten, Schüttvolumen, Löslichkeit etc, weniger günstig als bei einem Pulver, das in einem Düsenturm hergestellt wurde.

Es hat sich gezeigt, daß man eine besonders gute Löslichkeit und Stabilität des Sprühprodukts erhält, wenn man während des Sprühprozesses kontinuierlich ca. 15 bis 30 % des bereits gtrockneten Pulvers erneut dem Sprühprozeß im Bereich der Sprühdüsen einspeist. Durch diese Pulverrückführung läßt sich im Turm eine Instantisierung durchführen, welche die genannten Eigenschaften des Produkts positiv beeinflußt.

Die erfindungsgemäß erhältliche bzw. die nach dem erfindungsgemäßen Verfahren hergestellte Nahrungsgrundlage enthält bis auf das Phenylalanin alle wesentlichen Nährstoffe und stellt somit ein komplettes Produkt dar.

Als Quelle für die Aminosäuren finden keine Hydrolysate Anwendung, wie das beispielsweise bei den bisher bekannten Spezialerzeugnissen der Fall ist. Derartige auf Hydrolysaten beruhende Erzeugnisse enthalten jedoch immer noch Restmengen an Phenylalanin, die bei der Berechnung der Tagesdiät berücksichtigt werden müssen.

Die erfindungsgemäße Nahrungsgrundlage stellt außerdem ein Produkt dar, das hypoallergen bzw. hypoantigen ist, da allenfalls Dipeptide als Stickstoffbasis verwendet werden. Somit eignen sich die mit Hilfe der erfindungsgemäßen Nahrungsgrundlage hergestellten Nahrungen auch zur hypoallergenen Ernährung im Säuglingsalter (für Atopiker mit Phenylketonurie).

Die herkömmlichen Präparate auf Basis einer Aminosäurenmischung lassen sich nur schwer instantisieren und neigen zum Entmischen. Zudem haben derartige Präparate die Eigenschaft, mit Zuckerzusätzen zu bräunen, wodurch die Haltbarkeit eingeschränkt sein kann.

Auch müssen die bekannten Präparate bzw. Spezialerzeugnisse mit Pflanzenölen, Wasser und den übrigen Zutaten für die Diät gemischt werden. Dies führt zu einer sehr groben Emulsion des Fettes, so daß das Pflanzenöl nach einiger Zeit aufrahmt. Die Nahrung kann somit durch Schütteln nur in relativ größere Fettropfen zerkleinert werden.

Überraschenderweise hat sich nun gezeigt, daß die erfindungsgemäß erhältliche bzw. nach dem erfindungsgemäßen Verfahren erhaltene Nahrungsgrundlage bezüglich der Fettemulsion besonders stabil ist. Eine eventuell aufgerahmte trinkfertige Zubereitung läßt sich durch leichtes Schütteln wieder reemulgieren und zeichnet sich durch eine sehr gute Fettverteilung aus. Bedingt dadurch, daß die Fettröpfchen klein sind (kleiner als 5 µm) ist eine gute Verdaulichkeit gegeben.

Die erfindungsgemäße Nahrungsgrundlage stellt ein gut instantisiertes, rieselfähiges, gut wasserlösliches und homogenes, nicht entmischbares Pulver dar, das sich leicht dosieren läßt. Dieses Pulver läßt sich leicht in Wasser lösen und ist mindestens über 1h stabil. Schüttelt man dieses Produkt zusammen mit einer üblichen Säuglingsmilchnahrung an, dann bleibt es ebenfalls stabil und zeigt keine Phasentrennung in eine Wasser- und Fettphase.

Die erfindungsgemäße Nahrungsgrundlage läßt sich in beliebigen Mengenverhältnissen mit einer herkömmlichen Säuglingsmilchnahrung oder mit Muttermilch mischen. Die Handhabung ist einfach. Zur Bereitung der zu verfütternden Nahrung wird nur abgekochtes, warmes Wasser benötigt. Die Zubereitung der Diätnahrung durch den Arzt, die Klinikschwester oder die Mutter (oder auch den Vater) wird so erheblich vereinfacht.

Die erfindungsgemäße Nahrungsgrundlage wird von den Säuglingen und Kleinkindern wegen ihres verbesserten Geschmacks gut getrunken, so daß keine Verluste durch Spucken oder Nahrungsverweigerung entstehen. Diese Nahrungsgrundlage sättigt in gleicher Weise wie Säuglingsmilchnahrungen für altersentsprechende gesunde Kinder. Der Phenylalaninspiegel im Plasma wird im wünschenswerten Normbereich von 2 bis 4 mg/dl gehalten.

### Beispiel 1

### Herstellung einer phenylalaninfreien Säuglingsnahrungsgrundlage

Mit Ausnahme der Vitamine und einer geringen Laktosemenge für das Einbringen dieser Stoffe in das Endprodukt wird ein Sprühprodukt folgender Zusammensetzung hergestellt:

| | |
|---|---|
| L-Asparaginsäure | 0,502 % |
| L-Threonin | 0,551 % |
| L-Serin | 0,621 % |
| L-Lysin-L-Glutamat | 1,639 % |
| L-Glutaminsäure | 1,636 % |
| L-Prolin | 1,106 % |
| L-Glycin | 0,286 % |
| L-Alanin | 0,049 % |
| L-Valin | 0,816 % |
| L-Methionin | 0,286 % |
| L-Isoleucin | 0,694 % |
| L-Leucin | 1,167 % |
| L-Tyrosin | 0,694 % |
| L-Histidin | 0,286 % |
| L-Cystin | 0,286 % |
| L-Arginin | 0,408 % |
| L-Tryptophan | 0,204 % |
| L-Carnitin | 0,009 % |
| Taurin | 0,050 % |
| Magnesiumaspartat | 0,817 % |
| Cholinhydrogentartrat | 0,216 % |
| Kaliumcarbonat | 0,250 % |
| Natriumcarbonat | 0,069 % |
| Myo-Inosit | 0,010 % |
| Pflanzenfettmischung | 26,800 % |
| Emulgatoren (Monoglyceride, Lecithine) | 1,600 % |
| Stärke (gliadinfrei) | 7,000 % |
| Maltodextrine | 15,000 % |
| Laktose EuPH | 33,7797 % |
| Mineralstoffe (Kochsalz, Kaliumchlorid, Calciumcarbonat, Calciumglycerophosphat, di-Kaliumhydrogenphosphat) | 3,132 % |
| Spurenelemente (Eisen,- Kupfer-, Zink-, Mangan-, Jod-, Molybdän-, Chrom-, Fluor-Salze) | 0,0363 % |
| | 100,0000 % |

Zur Herstellung einer 100kg Charge schmilzt man 1,6 kg Emulgatoren bei 70 bis 75 °C in 26,8 kg einer Pflanzenfettmischung auf. Man bringt die Fett/Emulgatormischung unter intensivem Rühren (Ultraturrax, sowie Y-Stral) in 89 l heißes Wasser (70 bis 80°C) ein und verarbeitet zu einer Rohemulsion. Die Rührzeit sollte mindestens 5 Minuten betragen. Danach homogenisiert man die Rohemulsion mit einem Homogenisator mit einem Druck von 200 bar. Die homogenisierte Emulsion pumpt man in einen beheizbaren Doppelmanteltank. Danach arbeitet man unter Verwendung einer Venturi-Düse nacheinander folgende Rohstoffe unter intensivem Rühren ein:
Maltodextrin
Stärke
Laktose EP
Calciumglycerophosphat
Myo-Inosit
Cholinhydrogentartrat
Calciumcarbonat
di-Kaliumhydrogenphosphat
Kaliumchlorig
Natriumchlorid
Kaliumcarbonat
Natriumcarbonat
Kaliumjodid
Eisen-II-sulfat
Kupfer-II-sulfat
Zunksulfat
Mangansulfat
Natriummolybdat
Chrom-III-chlorid
Natriumfluorid
Taurin
L-Carnitin
Die Mineralstoffe Taurin und L-Carnitin löst man jeweils in den entsprechenden Volumina Wasser vor oder dispergiert, wobei die Wassermenge pro Substanz von der Löslichkeit und Konzentration der Substanz abhängt (nicht über 20 %). Man gibt die einzeln hergestellten Lösungen jeweils dem Ansatz zu. Nachdem man alle Kohlenhydrate und Mineralstoffe gelöst hat, setzt man die L-Aminosäuren in folgender Reihenfolge über die Venturi-Düse dem Ansatz zu und löst bzw. dispergiert unter intensivem Rühren:
L-Asparaginsäure
L-Threonin
L-Serin
L-Lysin-L-Glutamat
L-Glutaminsäure
L-Prolin
L-Glycin
L-Alanin
L-Valin
L-Methionin
L-Isoleucin
L-Leucin
L-Tyrosin
L-Histidin
L-Cystin
L-Arginin
L-Tryptophan
Magnesium-L-Aspartat
Der pH-Wert des erhaltenen Ansatzes sollte bei 6,1 bis 6,3 liegen. Falls notwendig, stellt man den pH-Wert des Ansatzes mit 10 %-iger wässriger Kaliumhydroxidlösung und/oder 10 %-iger wässriger Natriumhydroxidlösung und/oder 10 %-iger wässriger Natrium- oder Kaliumcarbonatlösung auf diesen pH von 6,1 bis 6,3 ein. Danach erwärmt man den Ansatz auf 70 bis 75 °C und homogenisiert mit einem Hochdruckhomogenisator.

| | |
|---|---|
| Stufe 1 | 100 - 150 bar |
| Stufe 2 | 50 - 70 bar |

Man erwärmt das homogenisierte Produkt in einem Schabeerhitzer auf 87 bis 93 °C, homogenisiert mit 100 bar nach und trocknet mit einem Sprühturm. Die Trocknung ist mit einer Sprühscheibe und mit Düsen möglich. Vorzugsweise trocknet man das Produkt mit Düsen.

| | | |
|---|---|---|
| Bedingungen: | Eingangstemperatur | 170 - 190 °C |
| | Ausgangstemperatur | 85 - 95 °C |
| | Düsendruck | 110 - 130 bar |
| | Restfeuchte | max. 2,5 % |
| | Pulverrückführung (Fließbett) | max. 25 °C |

Bringt man während des Sprühprozesses ca. 15 bis 30 % des getrockneten Pulvers erneut in den Sprühbereich des Turmes ein, dann kann man durch diese Pulverrückführung eine Instantisierung des Produktes erzielen.

Das auf die oben beschriebene Weise hergestellte Produkt ist gut rieselfähig und sehr gut in warmem Wasser löslich. Anschließend versetzt man das so hergestellte Sprühprodukt mit Vitaminen wie folgt:

| | |
|---|---|
| Sprühprodukt (Herstellung s.o.) | 98,0 % |
| Vitaminmischung | 0,6 % |
| Laktose EP | 1,4 % |
| | 100,0 % |

Man wiegt die Komponenten pro Charge ab und mischt 10 bis 15 Minuten in einem Taumelmischer. Anschließend kann man das Produkt verpacken. In Weißblechdosen, mit Stickstoff begast, hat das Produkt eine Haltbarkeit von mindestens zwei Jahren.

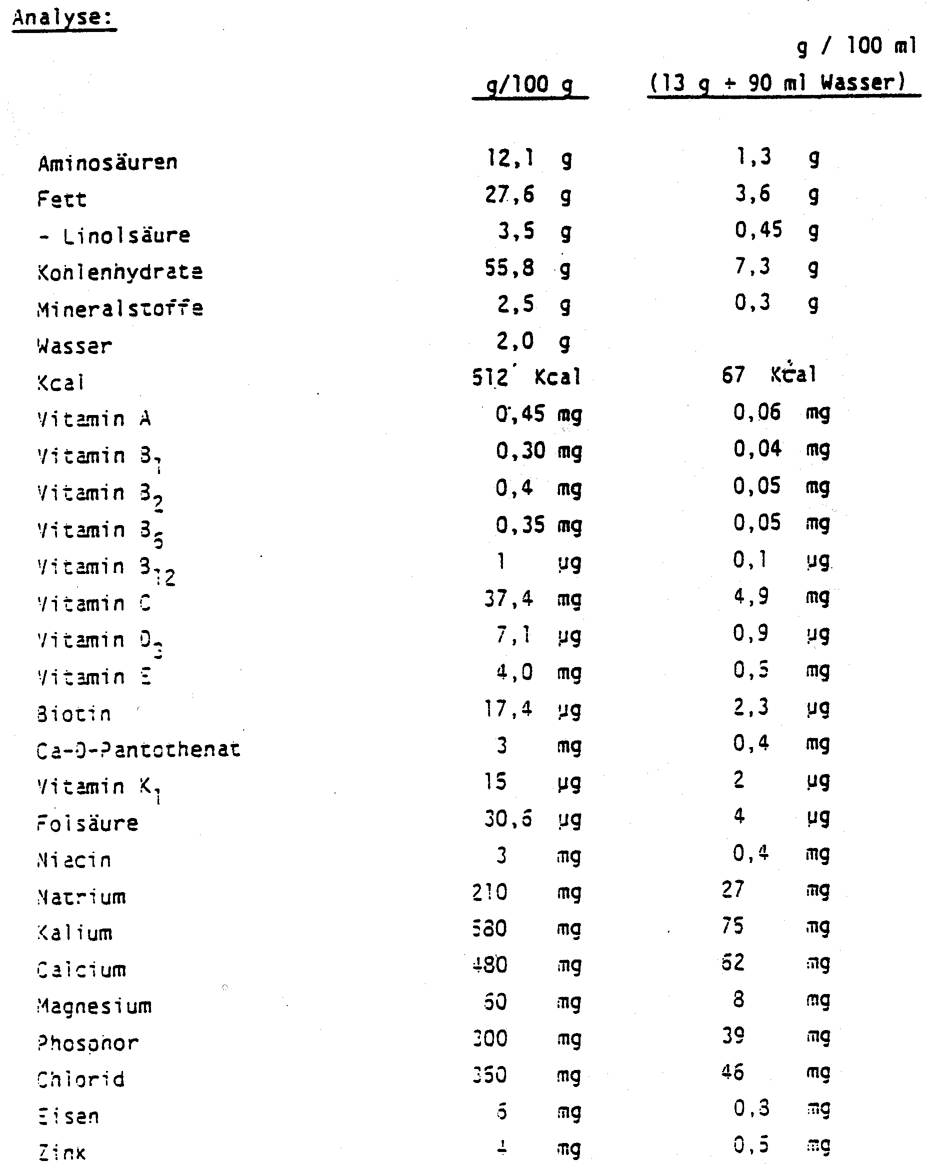

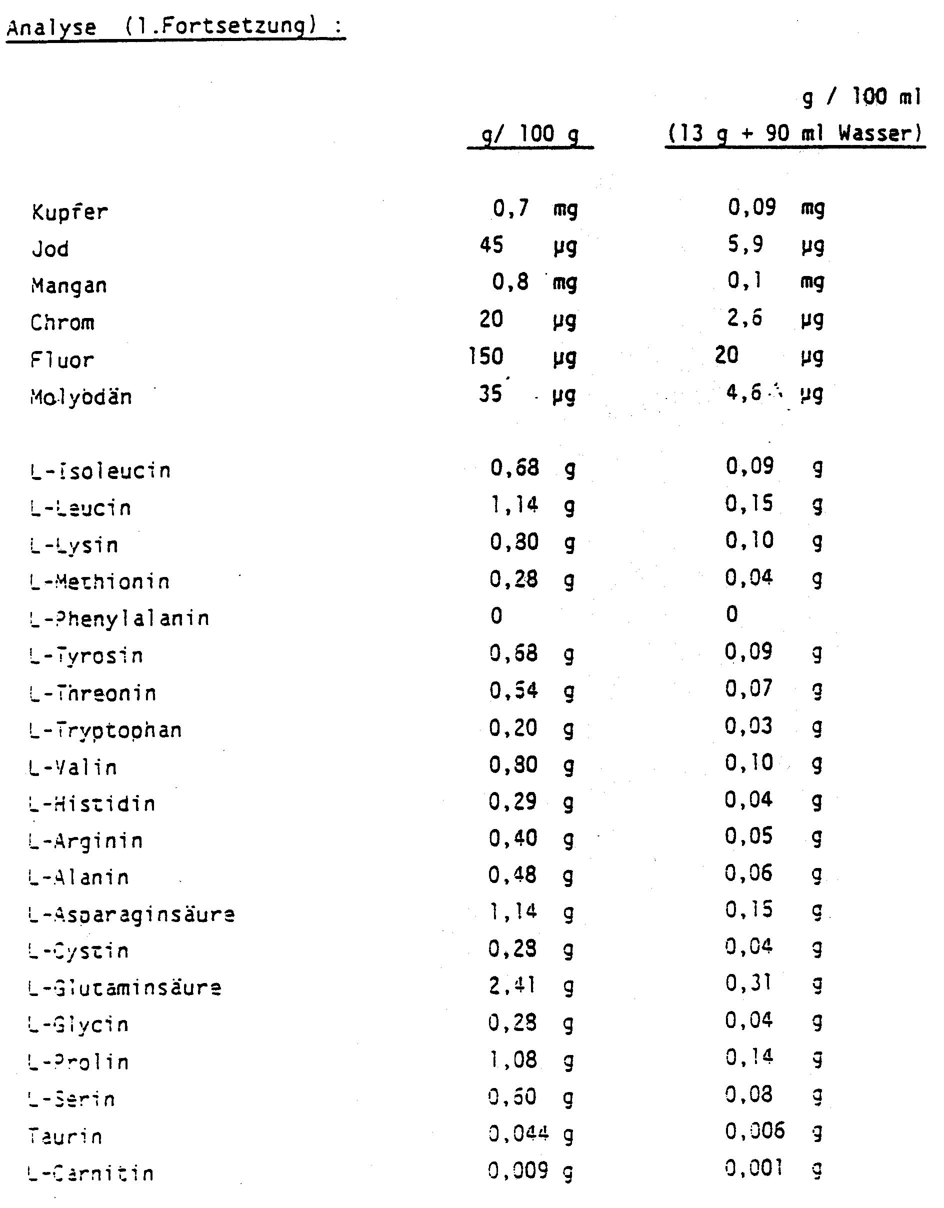

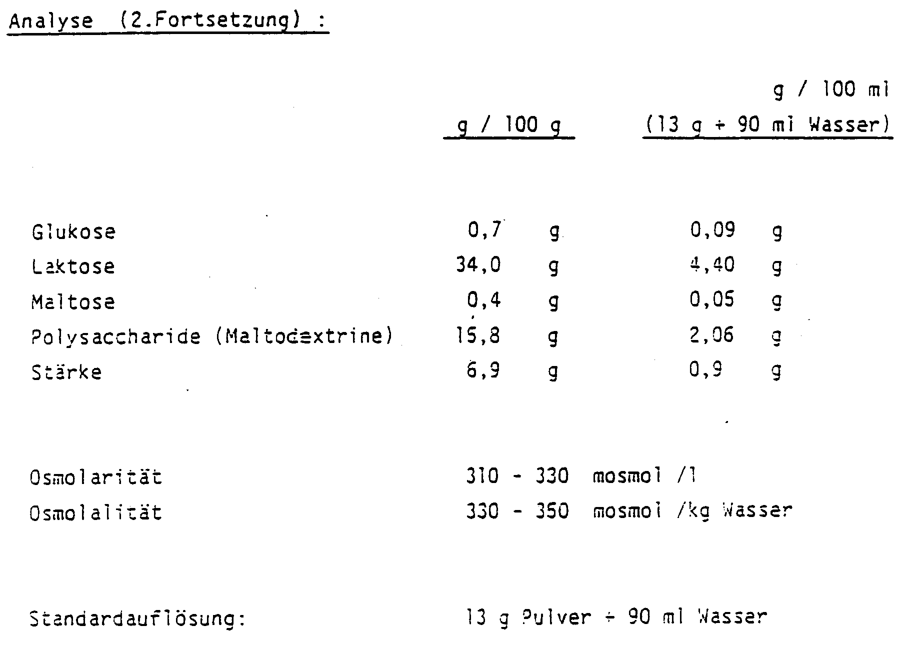

## Patentansprüche

1. Phenylalaninfreie Säuglings- und Kleinkindernahrungsgrundlage, die neben einer Aminosäurenmischung, die bis auf Phenylalanin alle für die Ernährung des Kindes erforderlichen L-Aminosäuren aufweist, auch Fett(e), Kohlenhydrate, Mineralstoffe und Spurenelemente enthält,
erhältlich durch
Sprühtrocknen eines die Aminosäuren, Fett(e), Kohlenhydrate, Spurenelemente und Mineralstoffe enthaltenden wässrigen Naßansatzes.

2. Nahrungsgrundlage nach Anspruch 1,
dadurch **gekennzeichnet**,
daß das Aminosäuremuster mit Ausnahme des Phenylalanins demjenigen in üblichen adaptierter Nahrungen und/oder in der Frauenmilch angeglichen ist.

3. Nahrungsgrundlage nach Anspruch 1 oder 2,
dadurch **gekennzeichnet**,
daß die Aminosäuren 5 - 20 %, die Fette 10 - 30 % und die Kohlenhydrate bis zu 70 % ausmachen, jeweils bezogen auf die Trockenmasse des Produkts, und daß die Mineralstoffe und Spurenelemente in solchen Mengen vorhanden sind, die den Empfehlungen und Richtlinien für Säuglings- und Kleinkindernahrungen entsprechen.

4. Nahrungsgrundlage nach einem der Ansprüche 1 bis 3,
dadurch **gekennzeichnet**,
daß sie die Aminosäuren vorwiegend als freie Säuren enthält.

5. Nahrungsgrundlage nach einem der Ansprüche 1 bis 4,
dadurch **erhältlich,**
daß man den Naßansatz vor dem Sprühtrocknen insbesondere auf 70 bis 80°C erhitzt, homogenisiert sowie ggf. pasteurisiert.

6. Nahrungsgrundlage nach Anspruch 5
dadurch **erhältlich,**
daß man zur Herstellung des Naßansatzes
a) zunächst eine Emulsion aus den Fetten und Emulgator(en) in Wasser herstellt und gegebenfalls homogenisiert,
b) alle Kohlenhydrate und anschließend alle Mineralstoffe und Spurenelemente in der gemäß a) erhaltenen Emulsion löst bzw. dispergiert und dann
c) alle Aminosäuren zusetzt,
wobei man vorzugsweise den pH-Wert des Naßansatzes auf mindestens 6,1 einstellt.

7. Nahrungsgrundlage nach Anspruch 5 oder 6,
dadurch **erhältlich,**
daß man die Sprühtrocknung in einem mit Düsen ausgestatteten Atomizer unter Trocknen und gleichzeitigem Agglomerieren durchführt.

8. Nahrungsgrundlage nach einem der vorhergehenden Ansprüche,
dadurch **gekennzeichnet**,
daß sie mit Vitaminen, insbesondere wasser- und fettlöslichen Vitaminen, versetzt ist.

9. Verfahren zur Herstellung einer Säuglings- und Kleinkindernahrungsgrundlage nach einem der Ansprüche 1 bis 7,
dadurch **gekennzeichnet**,
daß man einen die Aminosäuren, Fett(e), Kohlenhydrate, Spurenelemente und Mineralstoffe enthaltenden wässrigen Naßansatz sprühtrocknet.

10. Verfahren nach Anspruch 9,
dadurch **gekennzeichnet**,
daß man alle Bestandteile in Wasser löst/dispergiert bzw. emulgiert, den erhaltenen Naßansatz erhitzt (insbesondere auf 70 - 80 °C), homogenisiert, gegebenenfalls pasteurisiert und sprühtrocknet.

11. Verfahren nach Anspruch 9 oder 10,
dadurch **gekennzeichnet**,
daß man zur Herstellung des Naßansatzes
a) zunächst eine Emulsion aus den Fetten und Emulgator(en) in Wasser herstellt und gegebenfalls homogenisiert,
b) alle Kohlenhydrate und anschließend alle Mineralstoffe und Spurenelemente in der gemäß a) erhaltenen Emulsion löst bzw. dispergiert und dann
c) alle Aminosäuren zusetzt,
wobei man vorzugsweise den pH-Wert des Naßansatzes auf mindestens 6,1 einstellt.

12. Verfahren nach einem der Ansprüche 9 bis 11,
dadurch **gekennzeichnet**,
daß man die Sprühtrocknung in einem mit Düsen ausgestatteten Atomizer unter Trocknen und gleichzeitigem Agglomerieren durchführt und/oder daß man 15 bis 30 % des nach dem Spühtrocknen erhaltenen Pulvers erneut dem Spühprozess zuführt.

## Claims

1. Phenylalanine-free infant and small child nutritional base, which in addition to an amino acid mixture which, except for phenylalanine, has all L-amino acids necessary for the nutrition of the child, also contains fat(s), carbohydrates, mineral substances and trace elements,
obtainable by
spray-drying of an aqueous wet mixture containing the amino acids, fat(s), carbohydrates, trace elements and mineral substances.

2. Nutritional base according to claim 1, characterised in that the amino acid pattern, with the exception of the phenylalanine, is matched to that in normal adapted feeds and/or in human milk.

3. Nutritional base according to claim 1 or 2, characterised in that the amino acids constitute 5-20 %, the fats 10-30 % and the carbohydrates up to 70 %, each based on the dry weight of the product, and that the mineral substances and trace elements are present in amounts that correspond to the guidelines and recommendations for infant and small children's foods.

4. Nutritional base according to one of claims 1 to 3, characterised in that it contains the amino acids mainly as free acids.

5. Nutritional base according to one of claims 1 to 4, characterised in that before the spray-drying the wet mixture is heated in particular to 70 to 80°C, homogenised and optionally pasteurised.

6. Nutritional base according to claim 5, obtainable in that to prepare the wet mixture
a) firstly an emulsion of the fats and emulsifier(s) in water is prepared and optionally homogenised,
b) all carbohydrates and then all mineral substances and trace elements are dissolved or dispersed in the emulsion obtained according to a) and then
c) all amino acids are added,
the pH of the wet mixture preferably being adjusted to at least 6.1.

7. Nutritional base according to claim 5 or 6, obtainable in that the spray drying is performed in an atomiser equipped with nozzles, with drying and simultaneous agglomeration.

8. Nutritional base according to one of the foregoing claims, characterised in that it is mixed with vitamins, in particular water- and fat-soluble vitamins.

9. Process for the preparation of an infants and small children's nutritional base according to one of claims 1 to 7, characterised in that wet mixture containing the amino acids, fat(s), carbohydrates, trace elements and mineral substances is spray dried.

10. Process according to claim 9, characterised in that all components are dissolved/dispersed or emulsified in water and the wet mixture obtained is heated (especially to 70-80°C), homogenised, optionally pasteurised and spray-dried.

11. Process according to claim 9 or 10, characterised in that to prepare the wet mixture
a) firstly an emulsion of the fats and emulsifier(s) in water is prepared and optionally homogenised,
b) all carbohydrates and then all mineral substances and trace elements are dissolved or dispersed in the emulsion obtained according to a) and then
c) all amino acids are added,
the pH of the wet mixture preferably being adjusted to at least 6.1.

12. Process according to one of the claims 9 to 11, characterised in that the spray-drying is performed in an atomiser equipped with nozzles, with drying and simultaneous agglomeration, and/or that 15 to 30 % of the powder obtained after the spray-drying is again fed into the spray process.

## Revendications

1. Base d'alimentation sans phénylalanine pour nourrissons et petits enfants qui contient au côté d'un mélange d'acides aminés qui présente, à l'exception de la phénylalanine, tous les acides L-aminés nécessaires pour l'alimentation de l'enfant, également des corps gras, des carbohydrates, des substances minérales et des oligo-éléments que l'on peut obtenir par séchage par dispersion d'une charge humide aqueuse contenant un des acides aminés, des corps gras, des carbohydrates, des oligo-éléments et des substances minérales.

2. Base d'alimentation selon la revendication 1 caractérisée en ce que la composition en acides aminés, à l'exception de la phénylalanine, est adaptée à celle des aliments adaptés usuels et/ou du lait maternel.

3. Base d'alimentation selon la revendication 1 ou 2, caractérisée en ce que les acides aminés représentent 5 à 20 %, les corps gras de 10 à 30 % et les carbohydrates jusqu'à 70 % chaque fois par rapport au résidu sec du produit et en ce que les substances minérales et les oligo-éléments sont présents en quantités telles qu'elles correspondent aux recommandations et aux directives sur l'alimentation de nourrissons et de petits enfants.

4. Base d'alimentation selon l'une des revendications 1 à 3, caractérisée en ce qu'elle contient les acides aminés principalement sous forme d'acides libres.

5. Base d'alimentation selon l'une des revendications 1 à 4 que l'on peut obtenir en ce que, avant dispersion, on chauffe la charge humide notamment à une température de 70 à 80°C, on homogénéise et éventuellement on pasteurise.

6. Base d'alimentation selon la revendication 5 que l'on peut obtenir pour la préparation de la charge humide, en ce que
a) on prépare d'abord une émulsion constituée de graisses et d'émulsifiants dans l'eau et éventuellement on homogénéise,
b) on dissout ou bien on disperse tous les carbohydrates et ensuite toutes les substances minérales et les oligo-éléments dans l'émulsion obtenue selon a) et ensuite
c) on ajoute tous les acides aminés
en établissant de préférence une valeur de pH de la charge humide à au moins 6,1.

7. Base d'alimentation selon la revendication 5 ou 6 que l'on peut obtenir en ce qu'on réalise le séchage par dispersion dans un atomiseur muni de tuyères, par séchage et par agglomération en même temps.

8. Base d'alimentation selon l'une des revendications précédentes, caractérisée en ce qu'on ajoute des vitamines, plus particulièrement des vitamines solubles dans l'eau et dans des graisses.

9. Procédé pour la préparation d'une base d'alimentation de nourrissons et de petits enfants selon l'une des revendications 1 à 7, caractérisé en ce qu'on sèche par pulvérisation une charge aqueuse contenant les acides aminés, les corps gras, les carbohydrates, les oligo-éléments et les substances minérales.

10. Procédé selon la revendication 9, caractérisé en ce qu'on dissout/disperse ou encore émulsionne tous les constituants dans l'eau, on chauffe la charge humide obtenue (plus particulièrement à 70 à 80°C), on homogénéise et éventuellement on pasteurise et on sèche par dispersion.

11. Procédé selon la revendication 9 ou 10, caractérisé en ce que, pour la préparation d'une charge humide,
a) on prépare d'abord une émulsion dans l'eau constituée de graisses et d'émulsifiants et éventuellement on homogénéise,
b) on dissout, ou bien on disperse tous les carbohydrates et ensuite toutes les substances minérales et les oligo-éléments dans l'émulsion obtenue selon a) et ensuite
c) on ajoute tous les acides aminés en établissant de préférence une valeur de pH de la charge humide à au moins 6,1.

12. Procédé selon l'une des revendications 9 à 11, caractérisé en ce qu'on réalise le séchage par pulvérisation dans un atomiseur équipé de tuyères en séchant et en agglomérant en même temps et/ou en ce qu'on recycle dans le processus de séchage par dispersion 15 à 30 % de la poudre obtenue par séchage par dispersion.
